# EUROPEAN PATENT APPLICATION

(11) **EP 3 533 437 A1**
(43) Date of publication of application: **04.09.2019**
(21) Application number: 17864417.5
(22) Date of filing: 26.10.2017
(51) Int. Cl.: A61K 8/36, A61K 8/14, A61K 8/37, A61K 8/64, A61K 8/73, A61K 8/81, A61Q 19/00

(54) **COMPOSITION CONTAINING LINOLEIC ACID**

(30) Priority: 28.10.2016 JP 2016211570
(71) Applicant: Sunstar Inc., Takatsuki-shi Osaka 569-1195 (JP)
(72) Inventor: YASUDI, Hidekazu, Takatsuki-shi Osaka 569-1044 (JP); KISHIDA, Satomi, Takatsuki-shi Osaka 569-1044 (JP); DOMOTO, Takahiro, Takatsuki-shi Osaka 569-1044 (JP); MITSUI, Tsukasa, Takatsuki-shi Osaka 569-1044 (JP)
(74) Representative: Müller-Boré & Partner Patentanwälte PartG mbB
(86) International application number: PCT/JP2017/038806
(87) International publication number: WO 2018/079688

(57) **Abstract**

This invention provides a composition with higher stability of linoleic acid and/or a derivative thereof over time.

More specifically, the invention provides a composition comprising a continuous phase, and multilayer liposomes dispersed in the continuous phase, the continuous phase comprising at least one member selected from the group consisting of carboxy vinyl polymer or a salt thereof and cellulosic polymer, the multilayer liposomes comprising a cationized polymer in their layers and encapsulating a linoleic acid and/or a derivative thereof.

## Description

### Technical Field

The present invention relates to a composition comprising a linoleic acid.

### Background Art

Polyunsaturated fatty acids, typified by linoleic acid, have a skin-whitening effect. It is a known technique to incorporate polyunsaturated fatty acids into compositions for external use (Non-patent Document 1).

However, polyunsaturated fatty acids are known to have poor stability over time, and the polyunsaturated fatty acid content of the compositions decreases over time. Furthermore, compositions blended with polyunsaturated fatty acids may undergo discoloration or develop an unpleasant odor.

Particularly in Japan, laws, regulations, etc., require compositions for external use containing a polyunsaturated fatty acid as an active ingredient to stably retain 90% or more of the polyunsaturated fatty acid based on the specified amount for at least three years after production. Therefore, the development of effective stabilization techniques for polyunsaturated fatty acids has been desired.

For example, the following techniques have been proposed to improve the stability over time of polyunsaturated fatty acids: a technique of incorporating eugenol, isoeugenol, vitamin K, or the like (Patent Document 1); a technique of incorporating esterified polyunsaturated fatty acids (Patent Documents 2 to 4); a technique of producing an aerosol comprising nitrogen gas and a content containing a highly unsaturated fatty acid-containing lipid (Patent Document 5); a technique of producing an emulsion in which a polyunsaturated fatty acid is mixed with a specific amount of a dispersing agent, such as vitamin E (Patent Document 6); and a technique of incorporating tocopherol and/or phytic acid in a continuous phase in which liposomes containing a polyunsaturated fatty acid are dispersed (Patent Document 7).

### Citation List

### Patent Document

Patent Document 1: JPS63-72654A
Patent Document 2: JP2007-126438A
Patent Document 3: JP2007-269683A
Patent Document 4: JP2007-269684A
Patent Document 5: JPH8-81326A
Patent Document 6: JP2010-502733A
Patent Document 7: International publication WO2012/102364

### Non-patent Document

Non-patent Document 1: Monthly Book Derma, 98, 67-74, 2005

### Summary of Invention

### Problem to be Solved by the Invention

The present invention was made in view of the aforementioned current state of the prior art, and an object of the present invention is to provide a composition comprising a linoleic acid and/or a derivative thereof with higher stability over time.

### Means for Solving the Problem

As a result of extensive research to solve the above problems, the present inventors surprisingly found that the stability of linoleic acid and/or derivatives thereof over time can be significantly increased by encapsulating a linoleic acid and/or a derivative thereof into specific liposomes, and dispersing the liposomes in a continuous phase (dispersion medium) containing a specific polymer compound. The present inventors found a possibility of further improvements based on such a finding, and completed the present invention.

The present invention includes the main subjects shown below.

Item 1. A composition comprising a continuous phase, and multilayer liposomes dispersed in the continuous phase, the continuous phase comprising at least one water-soluble polymer selected from the group consisting of carboxy vinyl polymer or a salt thereof and cellulosic polymer, the multilayer liposomes comprising a cationized plant protein in their layers and encapsulating a linoleic acid and/or a derivative thereof.
Item 2. The composition according to Item 1, wherein the cellulosic polymer is at least one member selected from the group consisting of hydroxyethyl cellulose, hydroxypropyl cellulose, cellulose gum, and methyl cellulose.
Item 3. The composition according to Item 1 or 2, wherein the cationized plant protein has a form represented by the formula below:

   R^{p}-N⁺R¹R²R³

   wherein RP represents a plant protein, R¹ and R² are the same or different and each represent a C₁₋₆ alkyl group, and R³ represents a C₁₀₋₁₈ alkyl group.
Item 4. The composition according to any one of Items 1 to 3, wherein the plant protein is a hydrolyzed and/or hydroxypropylated plant protein.
Item 5. The composition according to any one of Items 1 to 4, wherein the plant protein is at least one member selected from the group consisting of wheat protein, rice protein, and soy protein.
Item 6. The composition according to any one of Items 1 to 5, wherein the cationized plant protein is a lauryldimonium hydroxypropyl hydrolyzed soy protein, or a cocodimonium hydroxypropyl hydrolyzed soy protein.
Item 7. The composition according to any one of Items 1 to 6, wherein the mass ratio of the water-soluble polymer and the cationized plant protein is 1:0.05 to 10.
Item 8. The composition according to any one of Items 1 to 7, wherein the mass ratio of a phospholipid constituting the multilayer liposomes (preferably the phospholipid being at least one member selected from the group consisting of lecithin and lecithin derivatives) and the linoleic acid and/or a derivative thereof encapsulated in the multilayer liposomes is 1:0.1 to 0.5.
Item 9. The composition according to any one of Items 1 to 8, wherein the composition is a liquid composition.
Item 10. The composition according to any one of Items 1 to 9, wherein the composition is a composition for external use.
Item 11. A method for producing a composition with high stability of linoleic acid and/or a derivative thereof over time, comprising homogenizing a composition comprising a phospholipid, a cationized polymer, a linoleic acid and/or a derivative thereof, and at least one member selected from the group consisting of carboxy vinyl polymers and cellulosic polymers.
Item 12. A method for increasing stability of linoleic acid and/or a derivative thereof over time in a composition, comprising homogenizing a composition comprising a phospholipid, a cationized polymer, a linoleic acid and/or a derivative thereof, and at least one member selected from the group consisting of carboxy vinyl polymers and cellulosic polymers.

### Effect of the Invention

The present invention enables significant increase in stability over time of linoleic acid and/or derivatives thereof encapsulated in liposomes. This enables effective use of the skin-whitening effect, etc., of linoleic acid and/or derivatives thereof.

### Description of Embodiments

Embodiments of the present invention are explained below in detail.

The present invention encompasses a composition comprising a continuous phase, and multilayer liposomes dispersed in the continuous phase. In this specification, the composition may be referred to as "the composition of the present invention". The composition of the present invention is a dispersion system in which multilayer liposomes are dispersed in a continuous phase. More specifically, the continuous phase is a dispersion medium, and multilayer liposomes are a dispersoid.

The continuous phase in the composition of the present invention contains at least one water-soluble polymer selected from the group consisting of carboxy vinyl polymers or salts thereof and cellulosic polymers. By incorporating such water-soluble polymers into the continuous phase, it is possible to significantly increase the stability over time of linoleic acid and/or derivatives thereof.

Carboxy vinyl polymer is an acrylic acid-based water-soluble polymer, and may also be referred to as "carbomer" in the technical field to which the present invention pertains. Examples of the carboxy vinyl polymer used in the present invention include not only carboxy vinyl polymers, but also salts of carboxy vinyl polymers. Examples of the salts of carboxy vinyl polymers include sodium salts, potassium salts, calcium salts, (mono-, di-, or tri-)ethanolamine salts, and amino acid salts. More specifically, examples of the salts of carboxy vinyl polymers include calcium potassium carbomer, AMP-carbomer, AMPD-carbomer, potassium carbomer, sodium carbomer, TEA-carbomer, and carbomer arginine. Further, known carboxy vinyl polymers may be used as the carboxy vinyl polymer of the present invention without specific limitation of the viscosity, the molecular weight, etc., insofar as they are suitable for, for example, a thickener for cosmetic products. A suitable example includes a carboxy vinyl polymer having a viscosity (25°C) of about 13,000 to 35,000 mPa·s when it is made into a 0.2% aqueous solution. Carboxy vinyl polymers having such a viscosity are commercially available. For example, Carbopol 940 (Lubrizol Corporation) can be commercially obtained.

The cellulosic polymer to be used in the present invention is a polymer obtained by etherifying or esterifying the hydroxyl groups of cellulose, or salts thereof. Known cellulosic polymers may be used as the cellulosic polymers of the present invention without specific limitation of the viscosity, the molecular weight, etc., insofar as they are suitable for, for example, a thickener for cosmetic products.

Suitable examples include methyl cellulose, ethyl cellulose, carboxymethyl cellulose, hydroxyethyl cellulose (HEC), hydroxypropyl cellulose (HPC), hydroxypropylmethyl cellulose (HPMC), and cellulose gum. Among them, methyl cellulose, hydroxyethyl cellulose, hydroxypropyl cellulose, and cellulose gum are preferable in terms of their superior effects of improving the stability over time of linoleic acid and/or derivatives thereof.

The weight-average molecular weight of the cellulosic polymer is not particularly limited insofar as the effects of the present invention are ensured. A cellulosic polymer having a weight-average molecular weight of, for example, about 50000 to 2000000 g/mol, may be used. The weight-average molecular weight may be measured, for example, by gel permeation chromatography.

Further, the cellulosic polymer may be etherified or esterified. Further, the degree of etherification or esterification of the cellulosic polymer is not particularly limited insofar as the effects of the present invention are ensured.

The above carboxy vinyl polymers and cellulosic polymers can be used singly, or in a combination of two or more. Further, when a combination of two or more kinds is used, the proportion of each component is not particularly limited.

The content of the carboxy vinyl polymer and the cellulosic polymer in the composition of the present invention is not particularly limited; however, the content is preferably about 0.05 to 5 mass%, more preferably about 0.1 to 4 mass%, further preferably about 0.2 to 3 mass%, and particularly preferably about 0.3 to 2 mass% based on the entire amount of the composition of the present invention.

The multilayer liposomes in the composition of the present invention contain a linoleic acid and/or a derivative thereof encapsulated therein. The state where "a linoleic acid and/or a derivative thereof is encapsulated therein" designates a state where a linoleic acid and/or a derivative thereof is enclosed in each liposome.

The linoleic acid used in the present invention may be a linoleic acid in the form of a fatty acid, or a linoleate. Examples of linoleates include linoleic acid metal salts such as linoleic acid sodium salt, or linoleic acid potassium salt; linoleic acid amino acid salts such as linoleic acid arginine salt, or linoleic acid lysine salt; linoleic acid amine salts such as linoleic acid monoethanolamine salt or linoleic acid triethanolamine salt; and the like.

Further, in the present invention, derivatives of linoleic acid may also be used in place of or in addition to linoleic acid. Examples of the derivatives of linoleic acid include linoleic acid esters such as ethyl linoleate, isopropyl linoleate, isostearyl linoleate, oleyl linoleate, glyceryl linoleate, tocopheryl linoleate, retinyl linoleate, lanolin linoleate, polyglyceryl-10 linoleate, linoleic acid menthol ester or the like.

Further, the linoleic acid derivatives used in the present invention encompass those having "linoleic acid/linoleate" in their names in the list of the Japan Cosmetic Industry Association. Examples of such linoleate derivatives include tocopheryl linoleate/oleate, glyceryl citrate/lactate/linoleate/oleate, oleic/linoleic/linolenic polyglycerides, hydroxystearic/linolenic/linoleic polyglycerides, caprylic/capric/linoleic triglyceride, PEG-4 proline linoleate, dilinoleic acid, sodium dilinoleate, diisostearyl dimer dilinoleate, diisopropyl dimer dilinoleate, dimethiconol fluoroalcohol dilinoleic acid, PEG-20 hydrogenated dimer dilinoleate, PEG-30 hydrogenated dimer dilinoleate, PEG-40 hydrogenated dimer dilinoleate, PEG-60 hydrogenated dimer dilinoleate, PEG-80 hydrogenated dimer dilinoleate, diisostearyl dimer dilinoleate, diisostearoyl polyglyceryl-3 dimer dilinoleate, dioctyldodecyl dimer dilinoleate, dicetearyl dimer dilinoleate, dimer dilinoleyl dimer dilinoleate, bis-behenyl/isostearyl/phytosteryl dimer dilinoleyl dimer dilinoleate, bis-isostearyl dimer dilinoleyl dimer dilinoleate, triisostearoyl polyglyceryl-3 dimer dilinoleate, linoleamidopropyl dimethylamine dimer dilinoleate, hydrogenated castor oil dimer dilinoleate, phytosteryl isostearyl dimer dilinoleate, di-C20-40 alkyl dimer dilinoleate, octyldodecyl/PPG-3 myristyl ether dimer dilinoleate, behenyl/phytosteryl dimer dilinoleate, phytosteryl/isostearyl/cetyl/stearyl/behenyl dimer dilinoleate, sucrose polylinoleate, triisostearyl trilinoleate, PPG-26/dimer dilinoleate copolymer, polyglyceryl-2 isostearate/dimer dilinoleate copolymer, polyglyceryl-2 isononanoate/dimer dilinoleate copolymer, ethylenediamine/stearyl dimer dilinoleate copolymer, diethylene glycol/dimer dilinoleic acid copolymer, (diethylene glycol/hydrogenated dimer dilinoleic acid copolymer, diglycerin/dilinoleic acid/hydroxystearic acid copolymer, dilinoleic acid/ethylenediamine copolymer, dilinoleic acid/butanediol copolymer, dilinoleic acid/propanediol copolymer, polyglyceryl-10 dimer dilinoleate/stearate/hydroxystearate, IPDI/PEG-15 cocamine copolymer dimer dilinoleate, ethylenediamine/hydrogenated dimer dilinoleate copolymer bis-di-C14-18 alkyl amide, bis-dioctadecylamide dimer dilinoleic acid/ethylenediamine copolymer, isomerized linoleic acid, carnitine isomerized linoleate, potassium glutathione isomerized linoleate, stearyl dimer dilinoleate/PEG-3 myristyl ether, dimer dilinoleate (IPDI/PEG-15 soyamine) copolymer, tri(oleic acid/ linoleic acid) glyceryl, (mango butter dimer dilinoleyl esters/dimer dilinoleate) copolymer, and the like.

The content of the linoleic acid and/or derivative thereof in the composition of the present invention is not particularly limited; however, the content is preferably about 0.01 to 2 mass%, more preferably about 0.1 to 1.5 mass%, particularly preferably about 0.5 to 1.2 mass%, based on the entire amount of the composition of the present invention. The content of the linoleic acid and/or derivative thereof is preferably not less than 0.01 mass% so as to more efficiently obtain whitening effects when the composition of the present invention is used. Further, the content of the linoleic acid and/or derivative thereof is preferably not more than 2 mass% so as to reduce the risk that the composition will develop an unpleasant odor or undergo discoloration over time.

The multilayer liposome used in the present invention contains a phospholipid as the membrane component. More specifically, the multilayer liposome used in the present invention is a lipid complex having a plurality of layers that are formed by self-assembly of a membrane component including a phospholipid. In particular, those containing lecithin as a phospholipid as a constituent (in particular, as a membrane component) are preferable. The lecithin is not particularly limited. Examples of lecithin include soybean lecithin, corn lecithin, cottonseed-oil lecithin, yolk lecithin, egg-white lecithin, and the like. Further, lecithin derivatives can also be used in place of or in addition to lecithin. Examples of lecithin derivatives include hydrogenated lecithin, and compounds obtained by introducing polyethylene glycol, aminoglycans, etc., into phospholipids of an aforementioned lecithin. Among these, soybean lecithin, yolk lecithin, and hydrogenated yolk lecithin are preferred; and soybean lecithin and yolk lecithin are particularly preferred, as the lethithin constituting the multilayer liposomes. Furthermore, a purified lecithin with a higher phospholipid purity (e.g., phosphatidylcholine, phosphatidylserine, phosphatidylethanolamine, phosphatidylinositol, phosphatidylglycerol, sphingomyelin, etc.) can also be used. These lecithins or lecithin derivatives can be used singly, or in a combination of two or more.

In the composition of the present invention, the mass ratio of the phospholipid and the linoleic acid and/or a derivative thereof is preferably 1:0.1 to 0.5, and more preferably 1:0.2 to 0.4.

Further, the content of the phospholipid is preferably about 0.1 to 10 mass%, more preferably about 0.2 to 7 mass%, and further preferably about 0.3 to 5 mass%, based on the entire amount of the composition.

Further, the multilayer liposome used in the present invention contains a cationized plant protein in the layers.

The cationized plant protein used in the present invention preferably has a form represented by the formula: RP-N⁺R¹R²R³, wherein RP represents plant protein, R¹ and R² are the same or different and each represent C₁₋₆ alkyl group, and R³ represents C₁₀₋₁₈ alkyl group.

In the formula above, examples of the plant protein represented by RP include wheat proteins, rice proteins, and soy proteins. Further, the plant protein represented by RP may be a hydrolyzed and/or hydroxypropylated plant protein. More specific preferable examples of the plant protein represented by RP include hydrolyzed wheat proteins, rice proteins, soy proteins, hydroxypropylated wheat proteins, rice proteins, soy proteins, and hydrolyzed and hydroxypropylated wheat proteins, rice proteins, soy proteins, and the like. When the plant protein represented by R^{p} is, for example, a hydroxypropylated protein, RP- can be expressed as R^{p1}-CH₂CH(OH)CH₂-(with R^{p1} representing a protein before hydroxypropylation). When the plant protein represented by R^{p} is a hydrolyzed and hydroxypropylated protein, RP can be expressed as R^{p2}-CH₂CH(OH)CH₂-(with R^{p2} representing a hydrolyzed protein residue).

Examples of the C₁₋₆ (1, 2, 3, 4, 5, or 6) alkyl group represented by R¹ and R² in the above formula include methyl, ethyl, propyl, isopropyl, and like linear or branched alkyl groups. Among them, methyl or ethyl is preferable.

Examples of the C₁₀₋₁₈ (10, 11, 12, 13, 14, 15, 16, 17, or 18) alkyl group represented by R³ in the above formula include decyl, lauryl, dodecyl, and like linear or branched alkyl groups. Examples include decyl, lauryl, dodecyl, and like alkyl groups. Among them, lauryl is preferable.

Preferable specific examples of cationized plant protein represented by the above formula R^{p}-N⁺R¹R²R³ include lauryldimonium hydroxypropyl hydrolyzed soy protein, lauryldimonium hydroxypropyl hydrolyzed wheat protein, steardimonium hydroxypropyl hydrolyzed wheat protein, cocodimonium hydroxypropyl hydrolyzed wheat protein, cocodimonium hydroxypropyl hydrolyzed rice protein, and cocodimonium hydroxypropyl hydrolyzed soy protein. Among them, lauryldimonium hydroxypropyl hydrolyzed soy protein or cocodimonium hydroxypropyl hydrolyzed soy protein is preferable.

The cationized plant protein preferably has an average molecular weight of, but not particularly limited to, about 500 to 5000.

In the composition of the present invention, the mass ratio of the water-soluble polymer (at least one water-soluble polymer selected from the group consisting of carboxy vinyl polymers and cellulosic polymers) and the cationized plant protein is preferably, but not particularly limited to, 1:0.05 to 10. The lower limit of the mass ratio may be 1:0.1 or more, 1:0.15 or more, 1:0.2 or more, or 1:0.25 or more, and the upper limit of the mass ratio may be 1:8 or less, 1:6 or less, 1:5 or less, or 1:4 or less. More specifically, for example, 1:0.1 to 8, 1:0.15 to 6, 1:0.2 to 5, or 1:0.25 to 4 is more preferable. The content of the cationized plant protein is about 0.01 to 10 mass%, more preferably about 0.02 to 7 mass%, and further preferably about 0.05 to 5 mass%, based on the entire amount of the composition. Further, the lower limit may be about 1, 2 or 3 mass%.

The composition of the present invention can be produced, for example, by first preparing a liposome dispersion in which multilayer liposomes encapsulating a linoleic acid and/or a derivative thereof are dispersed in a liquid dispersion medium (continuous phase), and then adding a carboxy vinyl polymer and/or a cellulosic polymer to the dispersion medium (continuous phase) of the liposome dispersion. Generally, water may be used as the dispersion medium of the liposome dispersion.

The method for producing the liposome dispersion is not particularly limited, and known methods can be used. Examples of such methods include (1) to (4) below.
(1) a method of first mixing a phospholipid (lecithin), a linoleic acid and/or a derivative thereof, and other components, and then hydrating the resulting mixture with an aqueous solution containing a pH adjuster, a polyhydric alcohol, sugars, etc., thereby forming liposomes;
(2) a method of dissolving a phospholipid (lecithin), a linoleic acid and/or a derivative thereof, and other components in an alcohol, polyhydric alcohol, or the like, and then hydrating the resulting mixture with an aqueous solution containing a pH adjuster, a polyhydric alcohol, sugars, etc., thereby forming liposomes;
(3) a method of forming a complex of a phospholipid (lecithin), a linoleic acid and/or a derivative thereof, and other components in water using ultrasonic waves, a French press, a homogenizer, or the like, thereby forming liposomes; and
(4) a method of mixing and dissolving a phospholipid (lecithin), a linoleic acid and/or a derivative thereof, and other components in ethanol, adding the resulting ethanol solution to a potassium chloride aqueous solution, and removing the ethanol, thereby forming liposomes.

Further, the composition of the present invention may also be produced by adding a carboxy vinyl polymer and/or a cellulosic polymer during the above-described preparation of the liposome dispersion. More specifically, it is possible to add a carboxy vinyl polymer and/or a cellulosic polymer at the time of dissolving a phospholipid or other components. For example, in methods (1) and (2) above, a carboxy vinyl polymer and/or a cellulosic polymer may be added to an aqueous solution before hydration; in method (3) above, a carboxy vinyl polymer and/or a cellulosic polymer may be added to a liquid to be treated by ultrasonic waves, a French press, or a homogenizer; and in method (4) above, a carboxy vinyl polymer and/or a cellulosic polymer may be added to the ethanol solution.

The above-described liposome dispersion may contain other polymers, proteins and hydrolysates thereof, mucopolysaccharides, etc., insofar as the effects of the present invention are not adversely affected. That is, the liposome dispersion may be produced by using other polymers, proteins and hydrolysates thereof, mucopolysaccharides, etc., as materials. They may be contained in the continuous phase. Examples of the polymers to be added to the liposome dispersion include, but are not limited to, sodium alginate and the like. The polymer content is about, but not particularly limited to, 0.001 to 20 mass%, preferably about 0.01 to 10 mass%, and particularly preferably about 0.05 to 5 mass%, based on the entire liposome dispersion. Examples of the protein and hydrolysate thereof include collagen, elastin, keratin, casein, and other like proteins; hydrolysates of these proteins; salts of the hydrolysates; esters of the hydrolysates; and enzyme-treated products of the hydrolysates. The content of the protein and hydrolysate thereof is, for example, about, but not particularly limited to, 0.001 to 5 mass%, and preferably about 0.01 to 1 mass%, based on the entire liposome dispersion. Examples of the mucopolysaccharides include chondroitin sulfate, hyaluronic acid, dermatan sulfate, heparan sulfate, mucoitinsulfuric acid, heparin and derivatives thereof, and salts thereof; among these, chondroitin sulfate, hyaluronic acid, and sodium salts thereof are particularly preferred. The mucopolysaccharide content is, for example, about, but not particularly limited to, 0.0005 to 5 mass%, and preferably about 0.001 to 1 mass%, based on the entire liposome dispersion.

Further, the continuous phase of the composition of the present invention may contain components other than the carboxy vinyl polymer and/or cellulosic polymer, insofar as the effects of the present invention are not impaired. For example, when the dispersion medium of the liposome dispersion is water, and when a poorly water-soluble substance (such as tocopherol) is used as one of the other components, a surfactant may be further added.

Preferred examples of the surfactants include nonionic surfactants, such as polyoxyethylene alkyl ether, polyoxyethylene fatty acid ester, polyoxyethylene sorbitan fatty acid ester, polyoxyethylene hydrogenated castor oil, and polyglycerol fatty acid ester surfactants. Preferred among them are hexaglycerol fatty acid esters and decaglycerol fatty acid esters, such as hexaglycerol monolaurate, hexaglycerol monomyristate, hexaglycerol monostearate, hexaglycerol monooleate, decaglycerol monolaurate, decaglycerol monooleate, decaglycerol monolinoleate, and decaglycerol monoisostearate. These surfactants can be used singly, or in a combination of two or more.

The composition of the present invention is preferably used as a composition for external use (a composition to be applied to the skin). Examples of compositions for external use include pharmaceutical compositions, quasi-drug compositions, and cosmetic compositions. It is particularly preferable to use the composition of the present invention as a composition for external use having a skin-whitening effect (i.e., a skin-whitening composition for external use) in the form of a drug, quasi drug, or cosmetic. The dosage form of the composition is not particularly limited. Examples of the dosage form include face packs, paste, ointment, cream, gel, lotion, emulsion, essence, face lotion, spray, and the like.

When the composition of the present invention is used as a composition for external use, the target of application is not particularly limited, but is preferably a human who wants to whiten the skin.

When the present invention is used as a composition for external use, components that can be generally used in the production of compositions for external use can also be used. Examples of such components include moisturizers, oil components, coloring agents, antioxidants, sequestering agent, preservatives, pH adjusters, refrigerants, flavoring agents, UV absorbing and scattering agents, antioxidants, medicinal properties, and the like. These components may be contained in the continuous phase.

Examples of moisturizers include polyols, such as propylene glycol, 1,3-butylene glycol, and glycerol; high-molecular-weight polysaccharides, plant extract, microorganism metabolites, and the like. These moisturizers can be used singly, or in a combination of two or more.

Examples of oil components include animal oil, vegetable oil, mineral oil, hydrocarbon oil, ester oil, waxes, higher alcohol, higher fatty acid, silicone oil and derivatives thereof, Vaseline, essential oil, and the like. These oil components can be used singly, or in a combination of two or more.

Furthermore, when the present invention is used as a composition for external use, components that can generally be contained in liposomes can also be used in the preparation of liposomes, within a range that does not impair the effect of the present invention. Examples thereof include antioxidants, such as ascorbic acid; organic acids, such as lactic acid and citric acid; lipids, such as phosphatidylglycerol and phosphatidylethanolamine; natural polymers, such as chitosan, fucoidan, and hyaluronic acid; synthetic polymers, such as polyethylene glycol; sugars, such as trehalose, lactulose, and maltitol; polyols, such as glycerol; and the like.

The present invention also includes a method for whitening skin (skin-whitening method) comprising the step of applying the composition of the present invention to the target skin. This method can be used, for example, for therapeutic and cosmetic purposes. That is, the skin-whitening method includes therapeutic and cosmetic methods for skin whitening. More specifically, the whitening method as mentioned herein can be used, for example, for the treatment of spots, dullness, melasma, post-inflammatory hyperpigmentation, etc., or for cosmetic purposes. The application target and application dose are as described above.

The present invention also includes a method for producing a composition with high stability of the linoleic acid and/or a derivative thereof over time, the method comprising homogenizing a composition comprising at least one member selected from the group consisting of carboxy vinyl polymers and cellulosic polymers, a phospholipid, a cationized plant protein, and a linoleic acid and/or a derivative thereof. This method is a method for producing the composition included in method (3) above.

Further, the present invention also includes a method for increasing the stability of the linoleic acid and/or a derivative thereof over time in a composition, the method comprising homogenizing a composition comprising at least one member selected from the group consisting of carboxy vinyl polymers and cellulosic polymers, a phospholipid, a cationized plant protein, and a linoleic acid and/or a derivative thereof.

The various conditions such as the components used in these methods are as described above. In these methods, it is possible to prepare a multilayer liposome containing a cationized polymer in the layer and encapsulating a linoleic acid and/or a derivative thereof by performing homogenization. This improves the stability of the linoleic acid and/or a derivative thereof encapsulated in the liposome over time.

### Examples

The present invention is more specifically described below in reference to Examples; however, the present invention is not limited to the following Examples.

### Production Example: Preparation of Composition

The compositions of Examples 1 to 5 and Comparative Examples 1 to 6 were prepared by mixing the various components shown in Table 1 below at proportions specified in Table 1, and treating the resulting mixtures with a homogenizer, thereby forming multilayer liposomes. The multilayer liposomes contained in the compositions of Examples 1 to 5 and Comparative Examples 4 to 6 contain cocodimonium hydroxypropyl hydrolyzed soy protein in their layers. The multilayer liposomes contained in the compositions of Comparative Examples 1 to 3 do not contain cocodimonium hydroxypropyl hydrolyzed soy protein in their layers. The average molecular weight of the cocodimonium hydroxypropyl hydrolyzed soy protein is about 1000.

### Test Example 1: Oxidation Acceleration Test

Each composition of Examples 1 to 5 and Comparative Examples 1 to 6 prepared in the above Production Example was made into a 10% aqueous solution using distilled water. Each aqueous solution was evenly mixed with 60 mM AAPH (2 2'-azobis(2-methylpropionamidine) dihydrochloride) at a mass ratio of 1:1. The resulting mixtures were reacted for 4 hours at 37°C, and cooled at 0°C. AAPH is a substance known as an oxidation promoter, which generates radicals by heat decomposition at elevated temperature. Further, by the reaction of the radicals with the linoleic acid contained in the liposome dispersion, oxidation is promoted.

Thereafter, quantitative determination of linoleic acid was performed using high-performance liquid chromatography, and the oxidation rate (%) of linoleic acid was calculated using the quantitative value of the linoleic acid after four-hour heating, based on the quantitative value of the linoleic acid before heating. A composition with a linoleic acid oxidation rate of less than 2.5% was evaluated as A; a composition with a linoleic acid oxidation rate of not less than 2.5% and less than 5.0% was rated as B; a composition with a linoleic acid oxidation rate of not less than 5.0% and less than 10.0% was rated as C; and a composition with a linoleic acid oxidation rate of 10.0% or more was rated as D. The column under the name of "Oxidation Acceleration Test" in Table 1 shows the linoleic acid oxidation rates and the evaluation results obtained in the above test.

As shown in Table 1, it was confirmed that the linoleic acid oxidation rates of the compositions of Examples 1 to 5 were all lower than those of the compositions of Comparative Examples 1 to 6. A comparison between each Example and Comparative Examples 1 to 3 revealed that oxidation of linoleic acid can be suppressed by encapsulating a linoleic acid in a multilayer liposome that contains a cationized plant protein in the layers. Further, a comparison between each Example and Comparative Examples 4 to 6 revealed that oxidation of linoleic acid can be suppressed by incorporating a cellulosic polymer or a carboxy vinyl polymer in the continuous phase.

### Test Example 2: Observation of exterior characteristics

After the preparation of the compositions of Examples 1 to 5 and Comparative Examples 1 to 6 in the above Production Example, the compositions were allowed to stand at 5°C for eight months. After eight months, the presence or absence of color change, the presence or absence of aggregation (as well as the size of the aggregated particles, if aggregation was observed), and the presence or absence of separation for each composition were confirmed by visual inspection compared with the state before being left, thereby evaluating the exterior characteristics of the compositions. The evaluation of exterior characteristics was performed using the following criteria.
▪ Color Change
   B: Slight color change was confirmed.
   C: Insignificant color change was confirmed.
   D: Color change was confirmed.
▪ Presence or Absence of Aggregation
   B: Aggregates were not confirmed, or aggregates with a very small particle diameter were confirmed.
   D: Aggregates with a large particle diameter were confirmed.
▪ Presence or Absence of Separation
   B: Separation was not confirmed.
   D: Separation was observed.

As is clear from Table 1 below, it was confirmed that, by incorporating a cellulosic polymer or a carboxy vinyl polymer in the continuous phase, almost no change in exterior characteristics was observed in the composition, even after the composition was stored for a long period of time.

The values of the components in Table 1 denote "mass%" unless otherwise specified.

**Table 1**

| Name of Components | | Example 1 | Example 2 | Example 3 | Example 4 | Example 5 | Comparative Example 1 | Comparative Example 2 | Comparative Example 3 | Comparative Example 4 | Comparative Example 5 | Comparative Example 6 |
|---|---|---|---|---|---|---|---|---|---|---|---|---|
| Soybean Lecithin | | 4 | 4 | 4 | 4 | 4 | 4 | 4 | 4 | 4 | 4 | 4 |
| Linoleic Acid | | 1.09 | 1.09 | 1.05 | 1.09 | 1.05 | 1.05 | 1.05 | 1.05 | 1.05 | 1.09 | 1.05 |
| Cocodimonium Hydroxypropyl Hydrolyzed Soy Protein | | 1 | 1 | 1 | 1 | 1 | | | | 1 | 1 | 1 |
| Water-soluble polymer | Cellulose Gum | 1 | | | | | | | | | | |
| | Methyl Cellulose | | 1 | | | | | | | | | |
| | Hydroxyethyl Cellulose | | | 1 | | | | 1 | | | | |
| | Hydroxypropyl Cellulose | | | | 1 | | | | | | | |
| | Carboxy Vinyl Polymer | | | | | 1 | | | 1 | | | |
| | Xanthan Gum | | | | | | 1 | | | 1 | | |
| | Guar Gum | | | | | | | | | | 1 | |
| | Cationized Guar Gum | | | | | | | | | | | 1 |
| Other components | 1,3-butylene Glycol | 15 | 15 | 15 | 15 | 15 | 15 | 15 | 15 | 15 | 15 | 15 |
| | Water-soluble Collagen | 0.2 | 0.5 | 0.5 | 0.5 | 0.5 | 0.5 | 0.5 | 0.5 | 0.5 | 0.5 | 0.5 |
| | Tocopherol | 0.1 | 0.1 | 0.1 | 0.1 | 0.1 | 0.1 | 0.1 | 0.1 | 0.1 | 0.1 | 0.1 |
| | Sodium Chondroitin Sulfate | 0.05 | 0.05 | 0.05 | 0.05 | 0.05 | 0.05 | 0.05 | 0.05 | 0.05 | 0.05 | 0.05 |
| | Methylparaben | 2 | 2 | 2 | 2 | 2 | 2 | 2 | 2 | 2 | 2 | 2 |
| | Ethylparaben | | | | | | | | | | | |
| | Propylparaben | | | | | | | | | | | |
| Water | | Balance | Balance | Balance | Balance | Balance | Balance | Balance | Balance | Balance | Balance | Balance |
| Total | | 100 | 100 | 100 | 100 | 100 | 100 | 100 | 100 | 100 | 100 | 100 |
| Oxidation Acceleration Test | Linoleic Acid Oxidation Rate (%) | 1.60 | 2.27 | 4.29 | 4.75 | 3.11 | 21.22 | 8.32 | 5.08 | 20.59 | 10.41 | 4.85 |
| | Evaluation | A | A | B | B | B | D | C | C | D | D | B |
| Exterior Characteristics | Color Change | B | B | B | B | B | B | C | B | C | C | D |
| | Presence or Absence of Aggregation | B | B | B | B | B | B | B | B | D | D | B |
| | Presence or Absence of Separation | B | B | B | B | B | B | B | B | B | D | B |

### Test Example 3: Oxidation Acceleration Test

The compositions of Examples A to D and Comparative Example A were prepared by mixing the various components shown in Table 2 below at proportions specified in Table 2, and treating the resulting mixtures with a homogenizer, thereby forming multilayer liposomes. The polyquaternium-64 is a cationized polymer (a quaternary ammonium salt polymer).

For the composition of each Example, the oxidation rate (%) of linoleic acid was evaluated in the same manner as in the oxidation acceleration test in Test Example 1. Table 2 shows the results. However, since the oxidation rate value varies in each study in this oxidation acceleration test, the obtained oxidation rate values were converted to the oxidation rate values in Table 1 based on the oxidation rate values in Example A having the same formulation as those of Example 3 in Table 1. More specifically, since the oxidation rate of Example 3 in Table 1 is 4.29 and the oxidation rate of Example A in Table 2 is 1.20, the oxidation rate of each Example shown in Table 2 is converted into the oxidation rates in Table 1 by multiplying the values by 4.29/1.20. For the converted oxidation rate values, as in the case above, an oxidation rate of less than 2.5% was evaluated as A, an oxidation rate of not less than 2.5% and less than 5.0% was rated as B, an oxidation rate of not less than 5.0% and less than 10.0% was rated as C, and an oxidation rate of 10.0% or more was rated as D. The evaluation results are summarized in Table 2.

**[Table 2]**

| Name of Components | | Same Formulation as that of Example 3 | Adjustment of Amount of Cationized Soy Protein (Adjustment of Content Ratio of Water-Soluble Polymer and Cationized Soy Protein) | | | Different Cationized Polymer was Used |
|---|---|---|---|---|---|---|
| | | Example A | Example B | Example C | Example D | Comparative Example A |
| Soybean Lecithin | | 4 | 4 | 4 | 4 | 4 |
| Linoleic Acid | | 1.05 | 1.05 | 1.05 | 1.05 | 1.05 |
| Cocodimonium Hydroxy Hydrolyzed Soy Protein | | 1 | 0.25 | 2 | 4 | |
| Polyquaternium-64 | | | | | | 1 |
| Water-soluble polymer | Hydroxyethyl Cellulose | 1 | 1 | 1 | 1 | 1 |
| Other components | 1,3-butylene Glycol | 15 | 15 | 15 | 15 | 15 |
| | Water-soluble Collagen | 0.5 | 0.5 | 0.5 | 0.5 | 0.5 |
| | Tocopherol | 0.1 | 0.1 | 0.1 | 0.1 | 0.1 |
| | Sodium Chondroitin Sulfate | 0.05 | 0.05 | 0.05 | 0.05 | 0.05 |
| | Methylparaben | 2 | 2 | 2 | 2 | 2 |
| | Ethylparaben | | | | | |
| | Propylparaben | | | | | |
| Water | | Balance | Balance | Balance | Balance | Balance |
| Total | | 100 | 100 | 100 | 100 | 100 |
| Linoleic Acid Content Ratio when Lecithin is assumed as 1 | | 0.25 | 0.25 | 0.25 | 0.25 | 0.25 |
| Cationized Component Content Ratio When Water-Soluble Polymer is Assumed as 1 | | 1 | 0.25 | 2 | 4 | 1 |
| Linoleic Acid Oxidation Rate (%) | | 1.20 | 1.32 | 1.13 | 1.00 | 1.92 |
| Conversion of Linoleic Acid Oxidation Rate Based on Oxidation Rate in Example 3 in Table 1 | | 4.29 | 4.72 | 4.04 | 3.58 | 6.86 |
| Evaluation | | B | B | B | B | C |

## Claims

1. A composition comprising a continuous phase, and multilayer liposomes dispersed in the continuous phase, the continuous phase comprising at least one water-soluble polymer selected from the group consisting of carboxy vinyl polymer or a salt thereof and cellulosic polymer, the multilayer liposomes comprising a cationized plant protein in their layers and encapsulating a linoleic acid and/or a derivative thereof.

2. The composition according to claim 1, wherein the cellulosic polymer is at least one member selected from the group consisting of hydroxyethyl cellulose, hydroxypropyl cellulose, cellulose gum, and methyl cellulose.

3. The composition according to claim 1 or 2, wherein the cationized plant protein has a form represented by the formula below:
R^{p}-N⁺R¹R²R³
wherein RP represents a plant protein, R¹ and R² are the same or different and each represent a C₁₋₆ alkyl group, and R³ represents a C₁₀₋₁₈ alkyl group.

4. The composition according to any one of claims 1 to 3, wherein the plant protein is a hydrolyzed and/or hydroxypropylated plant protein.

5. The composition according to any one of claims 1 to 4, wherein the plant protein is at least one member selected from the group consisting of wheat protein, rice protein, and soy protein.

6. The composition according to any one of claims 1 to 5, wherein the cationized plant protein is a lauryldimonium hydroxypropyl hydrolyzed soy protein, or a cocodimonium hydroxypropyl hydrolyzed soy protein.

7. The composition according to any one of claims 1 to 6, wherein the mass ratio of the water-soluble polymer and the cationized plant protein is 1:0.05 to 10.

8. The composition according to any one of claims 1 to 7, wherein the mass ratio of a phospholipid constituting the multilayer liposomes and the linoleic acid and/or a derivative thereof encapsulated in the multilayer liposomes is 1:0.1 to 0.5.

9. The composition according to any one of claims 1 to 8, wherein the composition is a liquid composition.

10. The composition according to any one of claims 1 to 9, wherein the composition is a composition for external use.

11. A method for producing a composition with high stability of linoleic acid and/or a derivative thereof over time, comprising homogenizing a composition comprising a phospholipid, a cationized polymer, a linoleic acid and/or a derivative thereof, and at least one member selected from the group consisting of carboxy vinyl polymers and cellulosic polymers.

12. A method for increasing stability of linoleic acid and/or a derivative thereof over time in a composition, comprising homogenizing a composition comprising a phospholipid, a cationized polymer, a linoleic acid and/or a derivative thereof, and at least one member selected from the group consisting of carboxy vinyl polymers and cellulosic polymers.
